# EUROPEAN PATENT SPECIFICATION

(11) **EP 2 479 166 B1**
(45) Date of publication and mention of the grant of the patent: **20.08.2014**
(21) Application number: 12162558.6
(22) Date of filing: 25.01.2010
(51) Int. Cl.: C07D 207/20, C07D 211/70, C07D 213/61

(54) **A process for the preparation of etoricoxib**
Verfahren zur Herstellung von Etoricoxib
Procédé de préparation d'étoricoxib

(30) Priority: 27.02.2009 IN MU04412009
(43) Date of publication of application: 25.07.2012
(62) Divisional of application: 10719784.0
(73) Proprietor: Cadila Healthcare Limited, Ahmedabad 380 015, Gujarat (IN)
(72) Inventor: Pandey, Bipin, 380 015 Gujarat (IN); Dave, Mayank Ghanshyambhai, 380 015 Gujarat (IN); Shah, Mitesh, 380 015 Gujarat (IN)
(74) Representative: Srinivasan, Ravi Chandran

(56) References cited:
- DAVIES I W ET AL: "A PRACTICAL SYNTHESIS OF A COX-2-SPECIFIC INHIBITOR", JOURNAL OF ORGANIC CHEMISTRY, AMERICAN CHEMICAL SOCIETY, EASTON.; US LNKD- DOI:10.1021/JO000870Z, vol. 65, 1 January 2000 (2000-01-01), pages 8415-8420, XP002326845, ISSN: 0022-3263

## Description

### FIELD OF INVENTION

The present invention relates to an improved process for the purification of Etoricoxib.

### BACKGROUND OF THE INVENTION

Etoricoxib is a selective COX-2 inhibitor which has been shown to be as effective as non-selective non-steroidal anti-inflammatory drugs in the management of chronic pain in rheumatoid arthritis, osteoarthritis and other COX-2 mediated disorders. Etoricoxib is 5-chloro-6'-methyl-3-[4-methylsulfonyl)phenyl]-2,3'-bipyridine having structural formula (I).

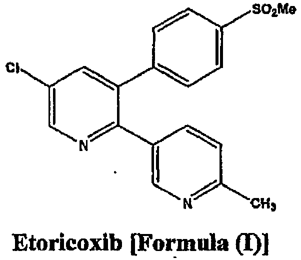

Etoricoxib belongs to a class of drugs known as COX-2 inhibitors that are used in the treatment of COX-2 mediated disorders. The therapeutic application of Etoricoxib as a COX-2 inhibitor is disclosed in WO 9610012 and WO 9616934.

Compounds of general formula (X) which includes Etoricoxib are disclosed in WO9803484 (US 5861419). Process for the preparation of Compound of formula (X) involves bromination of 2-amino pyridine derivative with bromine in acetic acid to provide the bromide of 2-amino pyridine derivative. Coupling of this bromide derivative with 4-(methylthio)phenyl-boronic acid is done in presence of a suitable base, which is then oxidized to give the corresponding sulphon. Then the amino group of sulphon is converted to corresponding halide. A second palladium catalyzed coupling of sulphon halide derivative with an appropriately substituted metal containing aromatic give compound of formula (X).

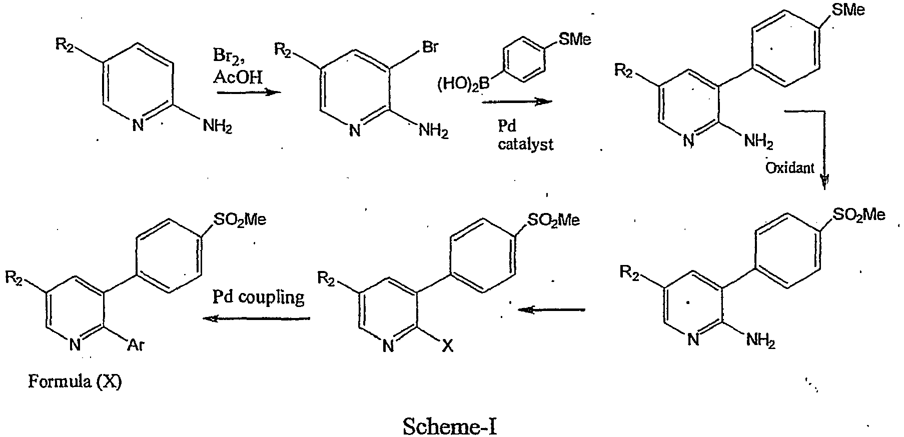

WO9803484 application also discloses a second method for the preparation of compound of formula (X), which is as per scheme II.

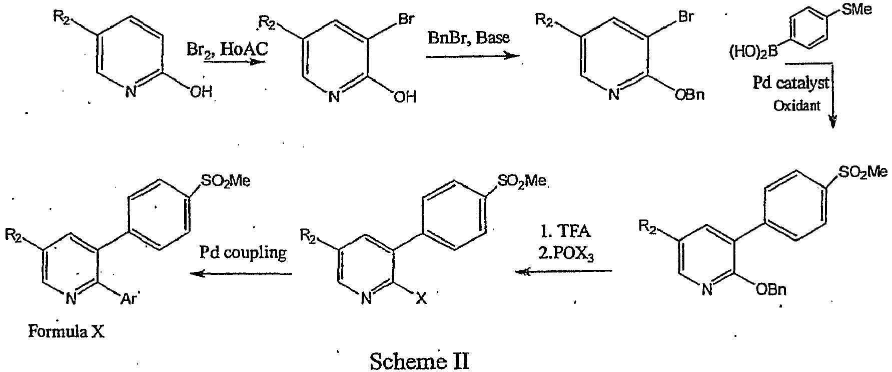

US 6812346 discloses a method for the preparation of Etoricoxib which comprises reacting a compound of formula B, wherein X is Br or Cl, with compound of formula C, wherein M is B(OH)₂ or SnMe₃, in the presence of a palladium catalyst to yield Etoricoxib.

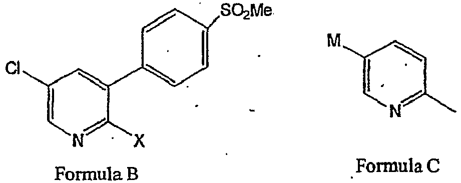

A process for the preparation of Etoricoxib as desribed in scheme III, is disclosed in US6040319, wherein X represents phosphates, sulfates, acetates, perchlorate, borates, antimonates, halides, benzoate, napsylate, particularly, hexafluorophosphate, sulfate, mesylate, tosylate, triflate, acetate, trifluoroacetate, tetrafluoroborate, tetraphenyl borate, hexafluoroantimonate, chloride, bromide, fluoride, iodide, benzolate and napsylate. Base used is Na and K hydroxide; Cs carbonate; Li, Na and K C₁₋₆ alkoxide; Li, Na and K amides, Li, Na and K hydrides.

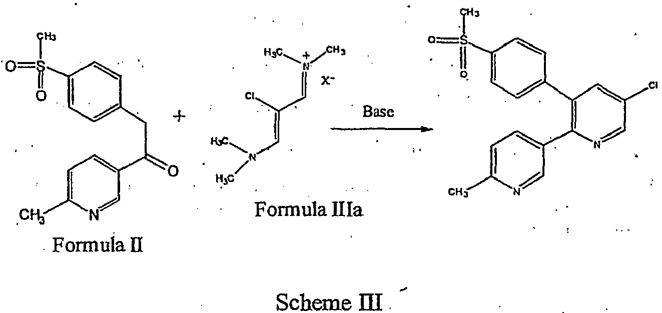

WO 0137833 discloses different polymorphs of Etoricoxib, specifically disclosed are forms II, III and IV, hemihydrate of form IV and sesquihydrate of form L WO 0192230 discloses polymorphic form V of Etoricoxib and process for its preparation. WO 0296877 discloses pharmaceutical composition comprising 10-50% of polymorphic form V and remainder of the compound comprising at least one polymorph selected from forms I, II, III and IV.

WO 2005085199 discloses different other polymorph of Etoricoxib, specifically disclosed forms IX, X, XI, XII, XIII, XIV, XV and XVI.
US 6252116 (divisional of US 6040319) discloses process for preparing intermediate of formula (III), particularly CDTH (2-chloro-1,3-bis(dimethylamino)trimethinium hexafluorophosphate) salt with hexafluorophosphate, tetraphenyl borate, hexafluoroantimonate as it counterion (X).

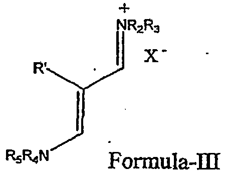

According to formula (III) R₂ through R₅ each independently represents C₁₋₆ alkyl, aryl or aralkyl group.

Certain heteroatom containing cycloalkyl substituted β-chlorovinamidinium salts are disclosed in J. Org. Chem., Vol: 66, No. 1, p. 251-255, 2001. But a large number of limitations are also reported. The method disclosed for the preparation of substituted β-chlorovinamidinium salt comprises, addition of chloroacetic acid to N-formyl compound containing a cycloalkyl group which optionally contains a heteroatom and the mixture was heated at 70 °C and then to it was added phosphorus oxychloride which after suitable work up gave the substituted β-chlorovinamidinium salt. This document also states that the β-chlorovinamidinium salt using N-formylmorpholine was obtained with very low yield (<10%) and pure sample could not be obtained by using this method.

Davies et al in J. Org. Chem., Vol. 65, January 2000, pages 8415 to 8420 disclose the synthesis of a COX-2-specific inhibitor via a ketosulfone intermediate.

The invention provides a process for the purification of Etoricoxib.

### BRIEF DESCRIPTION OF THE DRAWINGS

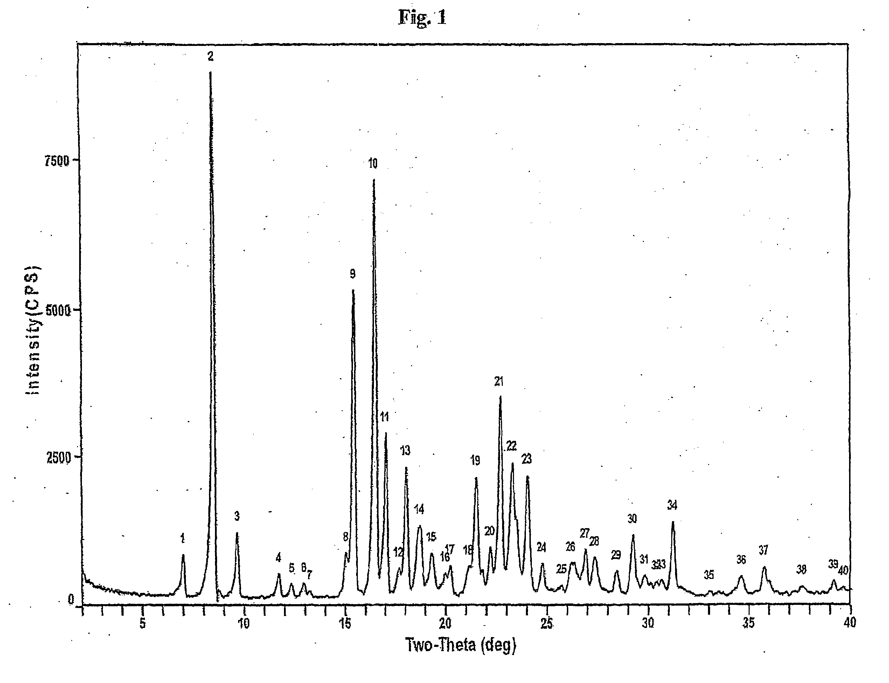

FIG.1 is a powder X-ray diffraction (XRPD) pattern of the Etoricoxib.

### OBJECTS OF THE INVENTION

The main objection of the present invention is to provide improved process for purifying Etoricoxib.

### DETAILED DESCRIPTION

As used herein, the term "THF" refers to tetrahydrofuran, the term "DMF" refers to dimethyl formamide, the term "DIPE" refers to di-isopropyl ether, the term "DMSO" refers to dimethyl sulphoxide, the term "MTBE" refers to methyl t-butyl ether, the term, "MEK" refers to methyl ethyl ketone, the term 'LHMDS' refers to lithium bis (trimethylsilyl)amide, the term 'KHMDS' refers to potassium bis (trimethylsilyl)amide.

Etoricoxib may be prepared by reacting substituted β-chlorovinamidinium salt of formula (IIIb) and a ketosulfone compound of formula (II). 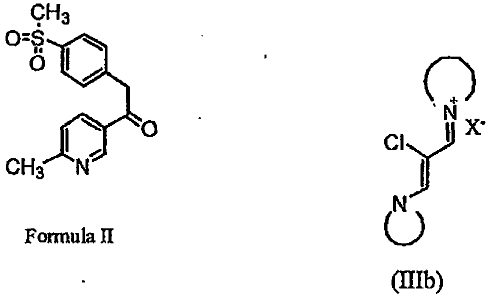 wherein 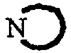 represents a cyclic group, optionally containing one or more heteroatom selected from N, O or S & 'X' represents the suitable counterion.

Preferably, the groups comprising 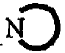 may be selected from morpholidinyl, peridinyl and a pyrolidinyl group.

The process of preparation of Etoricoxib does not form part of the present invention.

Etoricoxib, may be prepared by a process using the β-chlorovinamidinium salt of formula (IIIb). This process does not form part of the present invention. The process comprises the following steps:
(a) reacting a compound of formula-(IV) with suitable chloroacetyl halide or chloro acetic acid and phosphorous oxychloride to obtain the compound of formula (IIId) & further converting the compound of formula (IIId) into its suitable salt to obtain the compound of formula-(IIIb), as described above; and
(b) reacting compound of formula (IIIb) with compound of formula (II) in presence of suitable base and suitable solvent to obtain compound of formula (I).
The above process may be illustrated as per the following Scheme IV: 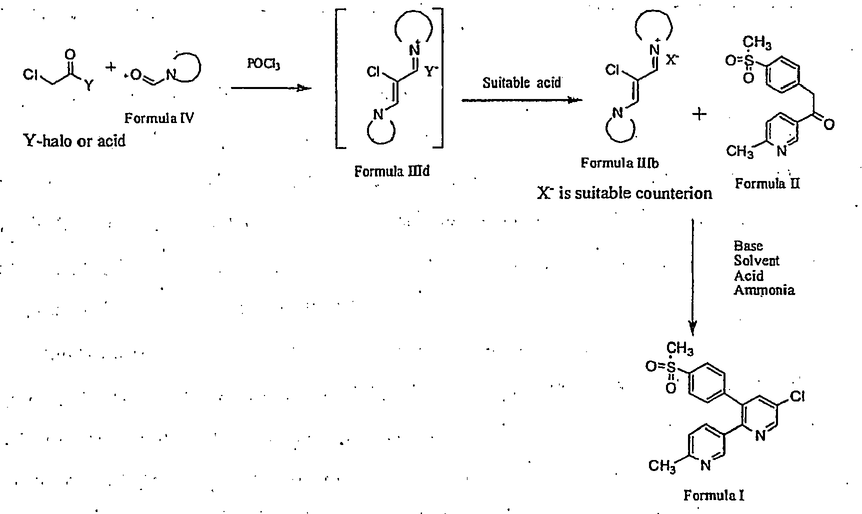 Preferably, the groups comprising 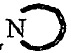 may be selected from morpholidinyl, piperidinyl and pyrolidinyl group.

Preferably, step (a) further comprises the preparation of compound of formula (IIIb) as described above.

Preferably, the β-chlorovinamidinium salt of formula (IIIb) is as described above. Preferably, step (a) comprises first combining chloroacetyl chloride and phosphorous oxychloride, and thereafter combining the N-formyl compound of formula (IV), to obtain the β-chlorovinamidinium salt of formula (IIIb), by processes as described above. Preferably, the temperature in step (a) is as described above.

Preferably, the reaction mixture is maintained in step (a) is as described above. Prior to step (b), the β-chlorovinamidinium salt of formula (IIIb) obtained in step (a) is separated. Preferably, the separation is done by filtration as described earlier in the specification.

The organic solvent in step (b) may be selected from the group comprising of suitable hydrocarbons such as benzene, toluene, xylene, ethyl benzene, trimethyl benzene & the like; suitable haloginated hydrocarbon solvents such as chloroform, dichloromethane, dichloroethane and the like or mixtures thereof; alcohols such as methanol, ethanol, t-butanol and like or mixtures thereof; ketones such as acetone; aprotic solvents such as DMF, dimethyl acetamide; ethers like diethyl ether, 1,4-dioxane, DIPE, MTBE, THF & the like, aprotic polar solvents such as DMF, DMSO, DMA; nitriles like acetonitriles or their suitable mixtures thereof.

The suitable base used in step (b) may be selected from suitable organic or inorganic bases such as hydroxides selected from NaOH, KOH & the like, carbonates such as NaHCO₃, Na₂CO₃, K₂CO₃, hydrides such as NaH and LHMDS, KHMDS and like; alkali metal alkoxides such as sodium, potassium, lithium t-butoxide and like; sodium, potassium, lithium isopropoxide and like.

The invention provides a process for purification of Etoricoxib (formula (I)).

Optionally, after completion of step (b) compound formula (I) can be isolated or subjected to insitu purification. Purification of compound formula (I) is done by using formalin solution and aq. ammonia in suitable solvent. The reaction mixture is stirred at suitable temperature and product is isolated using suitable solvent. Then after recrystallization is carried out using suitable solvent to obtain pure Etoricoxib.

The suitable solvent used for the purification of Etoricoxib may be selected from C₁-C₅ alcohol; ketones such as acetone and methyl ethyl ketone; ethers such as THF and dioxane or their suitable mixtures.

The suitable solvent used for the isolation of Etoricoxib may be selected from suitable alcohols or esters. The preferred solvent is isopropyl alcohol.

The suitable solvent used for recrystallization of Etoricoxib may be selected from suitable alcohols, esters, ethers or their suitable mixtures. The preferred solvent for recrystallisation is isopropyl alcohol.

In one of the embodiment of the invention is disclosed Etoricoxib with at least 99.9 % purity by HPLC.

Etoricoxib obtained according to the present invention is characterized by an XPRD pattern substantially in accordance with the pattern Fig. 1.

Etoricoxib obtained according to the present invention is also characterized by an XPRD peaks at about 7.00, 8.50, 9.67, 11.72, 12.35, 12.96, 13.24, 15.04, 15.44, 16.51, 17.06, 17.70, 18.07, 18.76, 19.34, 20.00, 20.24, 21.14, 21.50, 22.20, 22.70, 23.28, 24.02, 24.76, 25.73, 26.20, 26.90, 27.37, 28.44, 29.24, 29.83, 30.41, 30.66, 31.22, 33.04, 34.63, 35.76,37.62, 39.16,39.65 ° ± 0.2 ° degrees (20) (Fig.1) and has melting point in the range of 135-137 °C.

The invention is further described by the following examples, which are provided for illustration only and should not be construed to limit the scope of invention.

### Preparation Example 1

### Preparation of 2-chloro 1,3-(bis morpholinyl) trimethinium hexafluoro phosphate

Phosphorous oxychloride (6.6g) was added in to N-formyl morpholine (5g) at -5 to 10 °C and subsequently chloroacetyl chloride (5g) was added at room temperature and the reaction mixture was stirred. Further, N-formyl morpholine (5g) was added to the reaction mixture at room temperature and the reaction mass was heated at 55-110 °C for 3 to 24 hrs. The reaction mixture was then cooled to room temperature to obtain a semi solid mass, which was dissolved in dichloromethane and subsequently a solution of NaPF₆ was added at 0-10 °C over a period of one hour. Then into it 50 ml water was added, organic layer was separated, dried and concentrated till dry at less than 50 °C, to give 2-chloro 1,3-(bis morpholinyl) trimethinium hexafluoro phosphate. Yield=(4 g). HPLC purity = 44.63 %.

### Preparation Example 2

### Preparation of Etoricoxib

Potassium tert. butoxide (1.4g) was dissolved in THF and the solution was maintained at 5-25 °C for 10 minutes. Subsequently, a solution containing the 1-(6-methylpyridin-3-yl)-2[4-(methylsulfonyl)phenyl] ethanone (3g) in THF was added and kept this at 25-30 °C for 1. hr. 2-chloro 1,3-(bis morpholinyl) trimethinium hexafluoro phosphate of (formula III) (4g) in THF, obtained above, was added to the previously prepared solution of 1-(6-methylpyridin-3-yl)-2[4-(methylsulfonyl)phenyl] ethanone and the reaction mixture was heated at 30-55 °C for 2-24 hrs. The reaction mixture was dumped into a mixture of trifluoroacetic acid (0.6 g),' acetic acid (4.4g) and THF and further heated at 55-60 °C for 6-20 hrs after adding aqueous ammonia solution (30 ml) and ammonium acetate (0.8g). The reaction mixture was dumped into water and extracted with ethyl acetate and washed with 10% aqueous sodium bicarbonate solution. The organic layer was separated, dried & concentrated till dry at temperature less than 50 °C to obtain Etoricoxib. Yield = (3.1 g),
% purity by HPLC = 15.36 % (Etoricoxib), 64.15 % (unreacted ketosulfone compound of formula II

### Preparation example 3

### Preparation of 2-chloro 1,3-(bis piperidinyl) trimethinium hexafluorophosphate

Chloroacetic acid (4.75g, 0.05 mole) was added to N-formyl piperidine (13.5g, 0.119 mole) and the reaction mixture was heated to 70-75 °C to give a clear solution. Subsequently, phosphorous oxychloride (9.5 ml, 0.1 mole) was added over a period of 1-2 hrs and maintained at 70-75 °C for 3 hrs. The reaction mixture was cooled to room temperature and diluted with absolute alcohol (10 ml) and this reaction mass was added into the solution of NaPF₆. The reaction mass was stirred for 30 minutes at 0-10 °C, filtered and washed with water, to obtain 2-chloro 1,3-(bis piperidinyl) trimethinium hexafluoro phosphate (2 g), yield =10.42 %, HPLC purity- 83.63 %

### Preparation Example 4

### Preparation of 2-chloro 1,3-(bis piperidinyl) trimethinium hexafluoro phosphate

Chloroacetyl chloride (20g) solution was cooled and phosphorous oxychloride (27.2g) was added into it at -10 to 0 °C. Subsequently, N-formyl piperidine (40g) was added over a period of 1 hrs, maintained the reaction mass at 55-110°C for 6-20 hrs. Then the reaction mixture was cooled to room temperature and diluted with DMF (40 ml). This reaction mass was added into the solution of NaPF₆ over a period of 1 hr and the reaction mixture was stirred for 60 minute at 0-10 °C. The reaction mixture was filtered and washed with water to obtain crude 2-chloro 1,3-(bis piperidinyl) trimethinium hexafluoro phosphate (37.2 g), HPLC purity = 83.24 %.

Crude mass was purified by using methanol: water treatment to obtain 2-chloro 1,3-(bis piperidyl) trimethinium hexafluoro phosphate (18.8 g), yield = 27.48 %, HPLC purity = 99.19 %.

### Preparation Example-5

### Preparation of Etoricoxib

Potassium tert. butoxide (2.1g) was dissolved in THF (20ml) and the solution was maintained at 5-25 °C for 10 minutes. Subsequently, a solution of 1-(6-methylpyridin-3-yl)-2[4-(methylsulfonyl)phenyl] ethanone (5g) in THF was added and kept the mixture for 1hr at 25-30 °C. 2-chloro 1,3-(bis piperidyl) trimethinium hexafluoro phosphate salt of formula (IIIc) (8g), obtained in Example 4 above, in THF, was added to the previously prepared solution of ketosulfone and the reaction mixture was heated at 30-55 °C for 2-24 hrs. The reaction mixture was dumped into a mixture of trifloroacetic acid (1g), acetic acid (7.5g) and THF. The reaction mixture was further heated to 55-60 °C for 6-20 hrs, after adding ammonia solution (125 ml). Then cooled the reaction mixture to room temperature. The reaction mixture was dumped into water and extracted with ethyl acetate and washed with 10% aqueous sodium bicarbonate solution. The organic layer was separated and concentrated to dryness at less than 50 °C to give Etoricoxib 8.6 g.
HPLC purity- 91.28 % (Etoricoxib).
Crude mass was purified to obtain Etoricoxib (4.7g), yield = 75.9 %; HPLC purity = 96.31 %.

### Preparation Example-6

### Preparation of 2-chloro 1,3-(bis morpholinyl) trimethinium hexafluoro phosphate

Phosphorous oxychloride (6.6 g) was added in to N-formyl morpholine (5g) at a temperature of -5 to 5 °C. Subsequently chloroacetyl chloride (5g) was added at a temperature of 25-30 °C followed by addition of N-formyl morpholine (5g) at temperature 55 to 60 °C, there after the reaction mass was maintained at temperature 80-90 °C for 5 hr, & then cooled to 25-30 °C. The reaction mixture was diluted with dichloromethane 50 ml and the solution of NaPF₆ was added at 0-10 °C over a period of one hour. 50 ml water was added to the reaction mixture and organic layer was separated, dried, and concentrated to dryness at < 50 °C to obtain 2-chloro 1,3-(bis morpholinyl) trimetliinium hexafluoro phosphate.
% Yield = 23.13 %, (4 g), HPLC purity = 44.63 %.

### Preparation Example-7

### Preparation of Etoricoxib

Potassium tert. butoxide (1.4g) was dissolved in THF (7ml) and the solution was maintained at 25-30 °C for 10 minutes and then cooled to 10°C. Subsequently, solution of 1-(6-methylpyridin-3-yl)-2[4-(methylsulfonyl)phenyl] ethanone (5g) in THF was added and kept the mixture for 1hr at 25-30 °C. Subsequently, 2-chloro 1,3-(bis morpholinyl) trimethinium hexafluoro phosphate (4g) followed by THF (20ml) was added and the reaction mixture was maintained at 25-30 °C for 2 hrs. The reaction mixture was dumped in to the mixture of trifluoroacetic acid (0.6g), acetic acid (4.4g) and THF (10ml) and maintained the reaction mixture at 25-30 °C for 2 hrs. The reaction mixture was stirred at 55-60 °C for 3 hrs and aq. ammonia solution (30ml), and ammonium acetate (0.8g) was added and further stirred at 55-60 °C for 20 hrs. The reaction mixture was cooled to 25-30 °C then after water was added and extract with ethyl acetate (50ml). Organic layer was separated and washed with 10 % sodium bicarbonate solution, dried and concentrated to dryness < 50 °C to obtain Etoricoxib.
Yield = 3.1 g, (12.82 %), HPLC purity = 15.36.% (Etoricoxib), (unreacted 64.15 %-sulfone compound).

### Preparation Example-8

### Preparation of 2-chloro 1,3-(bis pyrolidinyl) trimethinium hexafluoro phosphate salt

Chloroacetyl chloride (5g) was cooled to -10 to 0 °C and phosphorous oxychloride (6.8g) was added in one lot at 0 to 10 °C, Subsequently, N-formyl pyrolidine (8.76g) was added drop wise at 0 to 5 °C and the reaction mixture was maintained at 90-100 °C for 6 hrs. There after the reaction mixture was cooled to 25-30 °C and diluted with DMF (10ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis pyrolidinyl) trimethinium hexafluoro phosphate salt. Yield = 8.7 g, HPLC purity = 93.09 %. Crude mass was purified using Methanol: water. Yield = 5.2 g, (32.9 %), HPLC purity = 98.87 %.

### Preparation Example-9

### Preparation of Etoricoxib

Potassium tert. butoxide (0.426g) was dissolved in THF (40ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (1g) in THF was added and kept the mixture for 1hr at 10-15 °C. Subsequently, 2-chloro 1,3-(bis pyrolidinyl) trimethinium hexafluoro phosphate salt (1.3g), in THF (8ml) was added and maintained the reaction mixture for 2 hrs at 25-30 °C. The reaction mixture was dumped in to the mixture of trifloroaceticacid (0.2g), acetic acid (1.5g), and THF (10ml) and maintained for 1 hour at 25-30 °C. Further the reaction mixture was heated at 60-70 °C for 15 hrs after aq. ammonia solution (25ml) was added and the reaction mixture was cooled to 25-30 °C. After then ethyl acetate and water was added, organic layer was separated and washed with water and 10 % sodium bicarbonate solution. Organic layer was separated, dried and concentrated to dryness < 50 °C to give crude Etoricoxib.
Yield = 0.9 g (40.84 %), % HPLC purity = 55.82 % (Etoricoxib), (unreacted sulfone compound = 34.44 %).

### Preparation Example-10

### Preparation of 2-chloro 1,3-(bis piperidinyl) trimethinium hexafluoro phosphate

Chloroacetyl chloride (5g) was cooled to -10 to 0 °C and phosphorous oxychloride (6.8g) was added in one lot at 0 to -10 °C. Subsequently, N-formyl piperidine (10g) was added drop wise at 0 to 5 °C and reaction mixture was maintained at 90-100 °C for 16 hrs. Thereafter reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
% Yield = 15.4 g (61.23 %), HPLC purity = 68.84 %.

### Preparation Example-11

### Preparation of 2-chloro 1,3-(bis piperidinyl) trimethinium hexafluoro phosphate

Phosphorous oxychloride (6.6g) was added in to N-formyl piperidine (6.1g) at temperature 15 to 17 °C. Subsequently chloroacetyl chloride (4.7g) was added drop wise at temperature 0-10 °C followed by addition of N-formyl piperidine (6.1g) in one lot at temperature 5 to 10 °C, then after the reaction mass was maintained at temperature 70-75 °C for 3 hr, cooled to 25-30 °C. The reaction mixture was diluted with DMF and the solution of NaPF₆ was added at 0-10 °C over a period of one hour. The reaction mixture was stirred for 60 minutes at 0-10 °C, filtered and washed with water, dried to obtain 2-chloro 1,3-(bis piperidinyl) trimethinium hexafluoro phosphate.
Yield **=3** g, (18.75 %), HPLC purity = 96.95 %

### Preparation Example-12

### Preparation of 2-chloro 1,3-(bis piperidinyl) trimethinium hexafluoro phosphate

N-formyl piperidine (30g) was cooled to 0 to 10 °C and Chloroacetyl chloride (30g) was added drop wise at 0 to 10 °C. Subsequently, phosphorous oxychloride (6.5g) was added drop wise at 0 to 10 °C. Further, N-formyl piperidine (30g) was added drop wise at 0-10 °C and reaction mixture was maintained at 80-90 °C for 16 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield =55.3 g, (54 %), HPLC purity = 72.74 %.

### Preparation Example-13

### Preparation of 2-chloro 1,3-(bis piperidinyl) trimethinium hexafluoro phosphate

Chloroacetyl chloride (10g) was cooled to 0 to 5 °C and N-formyl piperidine (10g) was added drop wise at 5 to 10°C and reaction mixture was stirred for 1 hr. Subsequently, phosphorous oxychloride (13.6g) was added drop wise at 0 to 10 °C and further N-formyl piperidine (10g) was added at 0-10 °C, stirred for 1 hrs at 0-5 ° C. The reaction mixture was maintained at 75-80 °C for 19 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 7.6 g, (22.22 %), HPLC purity = 80.33 %

### Preparation Example-14

### Preparation of 2-chloro 1,3-(bis piperidinyl) trimethinium hexafluoro phosphate

Chloroacetyl chloride (4.9g) was cooled to -5 to 0 °C and phosphorous oxychloride (6.7g) was added in one lot at 0 to -5 °C and stirred for 5minutes. Subsequently, N-formyl piperidine (8g) was added drop wise at 0 to 5 °C and further stirred for 10 minutes at 25-30 °C. The reaction mixture was maintained at 90-100 °C for 6 hrs. Then after reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with cold methanol: water to obtain 2-chloro 1,3-(bis piperidinyl) trimethinium hexafluoro phosphate salt.
Yield =10.5 g, (51.19 %), HPLC purity = 83.87 %.

### Preparation Example-15

### Preparation of 2-chloro 1,3-(bis piperidinyl) trimethinium hexafluoro phosphate

N-formyl piperidine (20g) was cooled to 0 to -5 °C and chloroacetyl chloride (10g) was added simultaneously, phosphorous oxychloride (13.4g) was added drop wise at -5 to 5°C and the reaction mixture was stirred at 25-30 °C for 10 minutes. The reaction mixture was maintained at 90-100 °C for 6 hrs. Then the reaction mixture was cooled to 25-30 °C and diluted with DMF. Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 20 g, (47.39 %), HPLC purity = 81.05 %.

### Preparation Example-16

### Preparation of 2-chloro 1,3-(bis piperidinyl) trimethinium hexafluoro phosphate

Chloroacetic acid (4.75 g, 0.05 mole) was added into N-formyl piperidine (13.5 g, 0.119 mole) at 25-30 °C and heat the reaction mixture at 70-75 °C. Subsequently phosphorous oxychloride (9.5 ml, 0.1 mole) was added over a period of 2 hrs at 70-75 °C. The reaction mixture was maintained at 70-80 °C for 3 hrs. Then after reaction mixture was cooled to 25-30 °C and diluted with absolute alcohol (10 ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 2 g, (10.42 %), HPLC purity = 83.63 %.

### Preparation Example-17

### Preparation of 2-chloro 1,3-(bis piperidinyl) trimethinium hexafluoro phosphate

Chloroacetyl chloride (20g) was cooled at -10 to 0 °C and phosphorous oxychloride (27.2g)was added in one lot at -10 to -15°C and simultaneously N-formyl piperidine (40g) was added drop wise at -8 to -10 °Cover a period of 1 hr. The reaction mixture was maintained at 90-100 °C for 6 hrs. Then after reaction mixture was cooled to 25-30 °C and diluted with absolute alcohol (10 ml). Subsequently, the reaction mass was added into the solution of NaPF₆ within 60 minutes at 0-10 °C and stirred for 60 minutes at 0-10 °C. The solid was precipitated out, which was filtered and washed with water to obtain 2-chloro 1,3 -(bis piperidinyl) trimethinium hexafluoro phosphate salt.
Yield = (45.27 %), 37.2 g, HPLC purity = 83.24 %.

### Prepration Example-18

### 1^{st} Purification of 2-chloro 1,3-(bis piperidinyl) trimethinium hexafluoro phosphate

2-chloro 1,3-(bis piperidyl) trimethinium hexafluoro phosphate (37g) was dissolved in (185ml) methanol and (18.5ml) water (5:0.5) at 25-30 °C. The reaction mixture was stirred at 70-75 °C for 1 hrs. The reaction mixture was cooled to 0-10 °C and further stirred for 1 hrs, reaction mass was filtered and washed with methanol: water (1:1).
Yield = 23 g, (74.67 %), HPLC purity = 97.25 %.

### Preparation Example-19

### 2^{nd} Purification of 2-chloro 1,3-(bis piperidinyl) trimethinium hexafluoro phosphate

2-chloro 1,3-(bis piperidyl) trimethinium hexafluoro phosphate (11.2g) was dissolved in (55ml) methanol and aq. solution of bisulphate at 25-30 ⁰C. The reaction mixture was stirred at 25-30 °C for 1 his, reaction mass was filtered and washed with water to obtain highly pure 2-chloro 1,3-(bis piperidinyl) trimethinium hexafluoro phosphate. Yield = 9.4 g, (83.92 %), HPLC purity = 99.19 %.

### Preparation Example-20

### Preparation of Etoricoxib

Potassium tert. butoxide (2.1g) was dissolved in THF (20ml) and the solution was maintained at 25-30 °C for 10 minutes and then cooled to 10-15 ⁰C. Subsequently, solution of 1-(6-methylpyridin-3-yl)-2[4-(methylsulfonyl)phenyl] ethanone (5g) in THF was added and kept the mixture for 1hr at 25-30 °C. Subsequently, 2-chloro 1,3-(bis piperidinyl) trimethinium hexafluoro phosphate salt (8g), in THF (40ml) was added and maintained the reaction mixture for 2 hrs at 25-30 °C. The reaction mixture was dumped in to the mixture of trifloroacetic acid (1g), acetic acid (7.5g), and THF (100ml) and maintained for 1 hour at 25-30 °C. Further the reaction mixture was heated at 55-65 °C for 17 hrs after aq. ammonia solution (125 ml) was added and the reaction mixture was cooled to 25-30 °C. After that ethyl acetate and water was added, organic layer was separated and washed with water and 10 % sodium bicarbonate solution. Organic layer was separated, dried and concentrated to dryness at less than 50°C to give crude Etoricoxib.
Yield = 8,6 g, HPLC purity = 91.28 % (Etoricoxib) (unreacted sulfone compound < 5 %).

### Preparation Example-21

### Preparation of Etoricoxib

Potassium tert. butoxide (11.6g) was dissolved in THF (90ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (27.5g) in THF was added and kept the mixture for 1hr at 15-25 °C. Subsequently, 2-chloro 1,3-(bis piperidinyl) trimethinium hexafluoro phosphate salt (44g), in THF (220ml) was added and maintained the reaction mixture for 3.5 hrs at 25-30 ⁰C. The reaction mixture was dumped in to the mixture of trifloxoaceticacid (5.5g), acetic acid (41.2g), and THF (65ml) and maintained for 1 hour at 25-30 °C. Further the reaction mixture was heated at 55-65 °C for 17 hrs after aq. ammonia solution (190ml) was added and the reaction mixture was cooled to 25-30 °C. Then ethyl acetate and water was added, organic layer was separated and washed with water and 10 % sodium bicarbonate solution. Organic layer was separated, dried and concentrated to dryness < 50 °C to give crude Etoricoxib.
Yield = 48.7 g, HPLC purity = 94.45 % (Etoricoxib), (unreacted sulfone compound < 5 %).

### Example-22

### 1^{st} Purification of Etoricoxib

(48.5 g) crude Etoricoxib (purity = 94.45 %, sulfone compound < 5%) obtained above was dissolved in 200 ml methanol and subsequently, (27.5g) formalin solution (37 %) and (27.5ml) aq. ammonia solution were added at 25-30 °C. The reaction mixture was stirred at 45-55 °C for 2 hrs and then remove the solvent under reduced pressure at < 55 °C. 250 ml ethyl acetate was added and reaction mixture was stirred. Organic layer was separated and washed with water, charcolized with (2.7g) charcoal, filtered and concentrated at < 55°C, azeotrope with 50 ml isopropyl alcohol to dryness.
Yield = 34.5 g (97.9 %), HPLC purity = 96.49 % (Etoricoxib), (unreacted sulfone compound < 1 %).

### Example-23

### 2^{nd} Purification of Etoricoxib

(34.4 g) crude Etoricoxib (purity = 96.49 %) was stirred in isopropyl alcohol at 60-65 °C for 1 hr. The reaction mixture was cooled to 25-30 °C and further stirred for 17 hrs. Further (27.5 ml) isopropyl alcohol was added and stirred for 10 minutes at 25-30 ⁰C, filter and washed with isopropyl alcohol, material was dried at 50-55 °C.
Yield = 23.3 g, (69.28 %), HPLC purity > 98 % Etoricoxib.

### Preparation Example-24

### Preparation of Etoricoxib

Potassium tert. butoxide (0.4g) was dissolved in DMF (4ml) and the solution was maintained at 25-30 °C for 10 minutes and then cooled to 10-15 °C. Subsequently, solution of 1-(6-methylpyridin-3-yl)-2[4-(methylsulfonyl)phenyl] ethanone (1g) in DMF was added and kept the mixture for 1hr at 15-25 °C. Subsequently, 2-chloro 1,3-(bis piperidinyl) trimethinium hexafluoro phosphate salt (1.6g), in DMF (16ml) was added and maintained the reaction mixture for 2 hr at 25-30 °C. The reaction mixture was dumped in to the mixture of trifloroacetic acid (0.2g), acetic acid (1.5g), and DMF (12ml) and maintained for 1 hour at 25-30 °C. Further the reaction mixture was heated at 55-65 °C for 18 hrs after ammonia solution (25ml) was added and the reaction mixture was cooled to 25-30 °C. After then ethyl acetate and water was added, organic layer was separated and washed with water and 10% sodium bicarbonate solution. Organic layer was separated, dried and concentrated to dryness < 50 °C to give crude Etoricoxib.
Yield = (55.12%) 1 g , HPLC purity = 67.8 % (Etoricoxib), (unreacted sulfone compound < 5 %).

### Example-25

### Preparation of Etoricoxib

1-(6-methylpyridin-3-yl)-2[4-(methylsulfonyl)phenyl] ethanone (9g) was dissolved in THF and the solution was maintained at 25-30 °C for 10 minutes and then cooled to 10-15 °C. Subsequently, potassium tert butoxide (4.1 g) was added and reaction mixture was maintained at 10-15 °C for 60 minutes. After then was added 2-chloro 1,3-(bis piperidinyl) trimethinium hexafluoro phosphate (14.8 g), followed by THF. The reaction mixture was maintained for 2 hrs at 10-15 °C and the reaction mixture was dumped into acetic acid (13 g) at 10-15 °C, washed with THF. Subsequently, the reaction mixture was maintained for 2 hour at 10-15 °C, further the reaction mixture was maintained at 60-65 °c for 16 hrs after adding aq. ammonia solution 25 ml and ammonium acetate (2.5 g). The reaction mixture was cooled to 25-30 °C, organic layer was separated and 25g aq. ammonia solution and 2 g formaldehyde solution was added at 25-30 °C. The reaction mixture was maintained at 60-65 °C for 1 hrs, cooled to 40 °C and 2.7 g sodium chloride was added in it & stirred for 30 minutes. Organic layer was separated and distilled till complete solvent removal under reduced pressure. The residue was azeotroped with toluene, and washed with water at 70-75 °C, aqueous layer was separated and extracted with toluene followed by toluene at 70-75 °C. Combined all the organic layers and washed with 7.5 % sodium bicarbonate solution followed by water. Further organic layer was separated and charcolized with 0.3 g charcoal at 60-65 °C, and separated organic layer was distilled under reduced pressure, residue was azeotroped with IPA at 25-30 °C. The reaction mixture was stirred at 75-80 °C for 30 minutes, cooled to 25-30 °C, further IPA was added at 25-30 °C, cooled to 10-15 °C, stirred for 2 hrs at 10-15 °C, filtered and washed with cold IPA.
Yield = 6.8 g (61 %), HPLC purity = 99.34 % (Etoricoxib), (unreacted sulfone compound < 5 %).

### Example-26

### Purification of Etoricoxib

Etoricoxib (5 g) (as obtained in example 25) was dissolved in isopropyl alcohol and stirred at 60-65 °C for 1 hrs after adding 0.17 g charcoal, filtered through hyflow & washed with 2 ml hot IPA. The reaction mixture was cooled to 25-30 °C and stirred for 2 hrs, filtered and washed with isopropyl alcohol, dried the material at 60-65 ⁰C to obtain pure Etoricoxib.
Yield = 3.6 g (72.87%), HPLC purity 99.83 % Etoricoxib.
Etoricoxib obtained is also characterized by an XPRD peaks at about 7.00, 8.50, 9.67, 11.72, 12.35, 12.96, 13.24, 15.04, 15.44, 16.51, 17.06, 17.70, 18.07, 18.76, 19.34,20.00, 20.24, 21.14, 21.50, 22.20, 22.70, 23.28, 24.02, 24.76, 25.73, 26.20, 26.90, 27.37, 28.44, 29.24, 29.83, 30.41, 30.66, 31.22, 33.04, 34.63, 35.76, 37.62, 39.16, 39.65° ± 0.2° degrees (2θ)(Fig.1) and has melting point in the range of 135-137 ⁰C.

### Preparation Example-27

### Preparation of 2-chloro 1,3-(bis piperidinyl) trimethinium hexafluoro phosphate

Chloroacetyl chloride (200g) was cooled at -10 to 0 °C and phosphorous oxychloride (271.5g) was added in one lot at 0 to -10 °C and simultaneously N-formyl piperidine (400g) was added drop wise at 0 to 10 °C over a period of 1 hr. The reaction mixture was maintained at 90-100 °C for 6 hrs. Then after reaction mixture was cooled to 25-30 °C and diluted with DMF. Subsequently, the reaction mass was added into the solution of NaPF₆ within 60 minutes at 0-10 ⁰C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 5.81.3 g (78.7 %), HPLC purity = 92.81 %.

### Preparation Example-28

### 1^{st} Purification of 2-chloro 1,3-(bis piperidinyl) trimethinium hexafluoro phosphate

2-chloro 1,3-(bis piperidinyl) trimethinium hexafluoro phosphate (580 g) (as obtained in example 27) was dissolved in a mixture of methanol and water (5:0.5) at 25-30 °C. The reaction mixture was stirred at 60-70 ⁰C for 1.5 hrs, cooled to 0-10 °C. Further, the reaction mixture was stirred for 1 hrs, filtered and washed with methanol: water (1:1) to obtain pure 2-chloro 1,3-(bis piperidinyl) trimethiniutn hexafluoro phosphate. Yield = 340.5 g (63.28 %), HPLC purity = 99.57 %.

### Preparation Example-29

### 2^{nd} Purification of 2-chloro 1,3-(bis piperidinyl) trimethinium hexafluoro phosphate

2-chloro 1,3-(bis piperidinyl) trimethinium hexafluoro phosphate (340.5g) was dissolved in methanol and aq. solution of bisulphate at 25-30 °C. The reaction mixture was stirred at 25-30 °C for 1 hr, reaction mass was filtered and washed with water to obtain highly pure 2-chloro 1,3-(bis piperidinyl) trimethinium hexafluoro phosphate.
Yield = 305 g, (90.15 %), HPLC purity = 99.65 %.
2-chloro 1,3-(bis piperidinyl) trimethinium hexafluoro phosphate obtained is also characterized by an XPRD peaks at about 9.061, 11.60, 12.06, 12.89, 13.64, 14.38, 16.022, 16.98, 17.56, 18.16, 19.43, 20.16, 20.46, 21.04, 21.48, 21.98, 22.25, 22.68, 23.35, 23.76, 24.24, 24.64, 25.92, 26.75, 27.42, 28.03, 28.55, 29.04, 29.68, 30.06, 30.52, 31.25, 31.96, 32.36, 33.31, 33.94, 34.30, 35.16, 35.80, 38.14, 39.47 ±.0.2 ° degrees (2θ)(Fig. 2, Form I) and has melting point in the range of 185-200 °C. (Decomposed).

### Preparation Example-30

### Preparation of 2-chloro 1,3-(bis piperidinyl) trimethinium hexafluoro phosphate

Chloroacetyl chloride (100g) was cooled to 0 °C and phosphorous oxychloride (135.7g) was added in one lot at 0 to -10°C and simultaneously N-formyl piperidine (200g) was added drop wise at 0 to 10 °C over a period of 1 hr. The reaction mixture was maintained at 90-100 °C for 6 hrs. Then the reaction mixture was cooled to 25-30 °C and diluted with DMF. Subsequently, the reaction mass was added into the solution of NaPF₆ (prepared by adding 5N aq. NaOH solution in aq. HPF₆ solution) within 60 minutes at 0-10 °C and stirred for 60 minutes at 0-10 °C. The solid was precipitated out, which was filtered and washed with water to obtain 2-chloro 1,3-(bis piperidinyl) trimethinium hexafluoro phosphate salt.
Yield = 189.3 g (45.9 5), HPLC purity: 82.41 %.

### Preparation Example-31

### 1^{st} Purification of 2-chloro 1,3-(bis piperidinyl) trimethinium hexafluoro phosphate

2-chloro 1,3-(bis piperidyl) trimethinium hexafluoro phosphate (180 g) (as obtained in example 30) was dissolved in a mixture of methanol and water (5:0.5) at 25-30 °C. The reaction mixture was stirred at 60-70 °C for 1.5 hrs, cooled to 0-10 °C. Further, the reaction mixture was stirred for 1 hr, filtered and washed with methanol: water (1:1) to obtain pure 2-chloro 1,3-(bis piperidinyl) trimethinium hexafluoro phosphate.
Yield =110 g (73.36 %), HPLC purity: 99.06 %

### Preparation Example-32

### 2^{nd} Purification of 2-chloro 1, 3-(bis piperidinyl) trimethinium hexafluoro phosphate

2-chloro 1,3-(bis piperidyl) trimethinium hexafluoro phosphate (109.6g; obtained in Example 31) was dissolved in methanol and aq. solution of bisulphate (29.5 g sodium bisulphite dissolved in 109.6 ml water) at 25-30 °C. The reaction mixture was stirred at 25-30 °C for 1 hr, reaction mass was filtered and washed with water to obtain highly pure 2-chloro 1,3-(bis piperidinyl) trimethinium hexafluoro phosphate.
Yield = 91.9 g (84.65 %), HPLC purity: 99.51 %.
2-chloro 1,3-(bis piperidinyl) trimethinium hexafluoro phosphate obtained is also characterized by an XPRD peaks at about 9.14, 10.142, 12.12, 12.96, 13.71, 17.06, 17.64, 18.24, 19.49, 20.520, 21.10, 21..56, 22.32, 22.74, 23.42, 24.32, 24.72, 26.05, 27.48, 28.10, 29.14, 29.79, 30.12,30.61, 31.30, 32.06, 32.43, 33.36, 34.02, 34.38 ° ± 0.2 ° degrees (2θ)( Fig. 3, Form II) and has melting point in the range of 190-197 °C.

## Claims

1. A process for purification of Etoricoxib comprising the steps of;
(a) treating Etoricoxib with formalin solution and aq. ammonia in suitable solvent;
(b) isolating the Etoricoxib with a suitable solvent;
(c) recrystallization of the solid obtained in step (b), from the solvent to obtain highly pure Etoricoxib.

2. The process as claimed in claim 1, wherein said suitable solvent used in step (a) is selected from C₁-C₅ alcohols, ketones, cyclic ethers or their suitable mixtures.

3. The process as claimed in claim 1, wherein suitable solvent used for isolation in step (b) is selected from C₁-C₅ alcohols.

4. The process as claimed in claim 1, wherein suitable solvent used for recrystallization in step (c) is selected from suitable alcohols.

5. The process as claimed in claim 1, wherein Etoricoxib obtained is with at least 99% purity by HPLC.

## Patentansprüche

1. Verfahren zur Reinigung von Etoricoxib, umfassend die Schritte:
(a) Behandeln von Etoricoxib mit Formalinlösung und wässrigem Ammoniak in einem geeigneten Lösungsmittel,
(b) Isolieren des Etoricoxibs mit einem geeigneten Lösungsmittel,
(c) Umkristallisieren des in Schritt (b) erhaltenen Feststoffs aus dem Lösungsmittel, um hochreines Etoricoxib zu erhalten.

2. Verfahren nach Anspruch 1, wobei das in Schritt (a) verwendete geeignete Lösungsmittel ausgewählt ist aus C₁-C₅-Alkoholen, Ketonen, cyclischen Ethern oder deren geeigneten Gemischen.

3. Verfahren nach Anspruch 1, wobei das für das Isolieren in Schritt (b) verwendete geeignete Lösungsmittel ausgewählt ist aus C₁-C₅-Alkoholen.

4. Verfahren nach Anspruch 1, wobei das für das Umkristallisieren in Schritt (c) verwendete geeignete Lösungsmittel ausgewählt ist aus geeigneten Alkoholen.

5. Verfahren nach Anspruch 1, wobei das erhaltene Etoricoxib in einer Reinheit von mindestens 99% gemäß HPLC vorliegt.

## Revendications

1. Procédé pour purifier de l'étoricoxib, comportant les étapes suivantes :
a) traiter de l'étoricoxib avec de la formaline et de l'ammoniaque, dans un solvant approprié,
b) isoler l'étoricoxib au moyen d'un solvant approprié,
c) et recristalliser dans le solvant le solide obtenu dans l'étape (b), de manière à obtenir de l'étoricoxib de haute pureté.

2. Procédé conforme à la revendication 1, dans lequel ledit solvant approprié utilisé dans l'étape (a) est choisi parmi les alcools en C₁-C₅, les cétones, les éthers cycliques et leurs mélanges appropriés.

3. Procédé conforme à la revendication 1, dans lequel ledit solvant approprié utilisé pour l'isolement dans l'étape (b) est choisi parmi les alcools en C₁-C₅.

4. Procédé conforme à la revendication 1, dans lequel le solvant approprié utilisé pour la recristallisation dans l'étape (c) est choisi parmi les alcools appropriés.

5. Procédé conforme à la revendication 1, dans lequel on obtient de l'étoricoxib doté d'une pureté, déterminée par CLHP, d'au moins 99 %.
